# EUROPÄISCHE PATENTANMELDUNG

(11) **EP 1 897 499 A2**
(43) Veröffentlichungstag der Anmeldung: **12.03.2008**
(21) Anmeldenummer: 07017517.9
(22) Anmeldetag: 06.09.2007
(51) Int. Cl.: A61B 10/00

(54) **Speichelsammler, Herstellungsverfahren und Sammelverfahren**

(30) Priorität: 07.09.2006 DE 102006042522
(71) Anmelder: Matallana-Kielmann, Michael, 72116 Mössingen (DE)
(72) Erfinder: Matallana-Kielmann, Michael, 72116 Mössingen (DE)
(74) Vertreter: Castell, Klaus

(57) **Zusammenfassung**

Die Erfindung betrifft ein Speichelsammler (1) zum Sammeln von Speichel für eine Diagnose anhand des Speichels, insbesondere zum Nachweis von diagnostischen Krankheiten, diagnostischen Stoffen, pharmakologischen Stoffen, diagnostischen Kontaminierungen, pharmakologischem Missbrauch von Stoffen und Drogenkonsum.

Herkömmliche Speichelsammler bestehen aus Teststreifen, wobei oft die erzeugte Speichelmenge beim Probanden für eine sichere Detektion nicht ausreicht.

Die Erfindung sieht vor, einen Duftstoffträger (7) für einen Duftstoff vorzusehen, der einen Probanden (9) zu erhöhtem Speichelfluss veranlasst.

## Beschreibung

Die Erfindung betrifft einen Speichelsammler für eine Diagnose anhand des Speichels, ein Verfahren zum Herstellen eines Speichelsammlers für eine Diagnose anhand des Speichels sowie ein Verfahren zum Sammeln von Speichel.

Es sind in vitro diagnostische Testsysteme bekannt, bei welchen unter Verwendung von Antikörpern und/oder Antigenen ein Nachweis von spezifischen Krankheiten oder Stoffen ermöglicht wird. Hierzu wird einem Probanden zunächst Speichel entnommen, um möglichst leicht die Diagnose durchführen zu können.

Die Speichelsammler sind in der Regel Teststreifen, auf denen ein Signal anhand von Antikörperreaktionen und/oder Antigenreaktionen abgelesen werden kann. Teilweise sind die streifenförmigen Speichelsammler in Kassetten eingebaut, um eine bessere Handhabung zu ermöglichen.

Die Hauptanwendungen dieser Testsysteme sind in der Detektion von Krankheiten und im Nachweis von bestimmten Stoffen im Speichel zu sehen. Häufig werden die Teststreifen eingesetzt, um eine schnelle Nachweismöglichkeit von pharmakologischem Konsum von Stoffen und deren Metaboliten zu ermöglichen. Unter Metaboliten ist zu verstehen, dass Stoffe während des Abbaus in einem physiologischem Prozess in Abbauprodukte umgesetzt werden.

Der Erfindung liegt die Aufgabe zu Grunde, die bestehende Technologie zu verbessern.

Diese Aufgabe löst nach einem ersten Aspekt der Erfindung ein Speichelsammler für eine Diagnose anhand des Speichels, wobei ein Duftstoffträger für einen Duftstoff vorgesehen ist, der einen Probanden zu erhöhtem Speichelfluss veranlasst.

Begrifflich sei hierzu erläutert, dass ein Duftstoff sowohl in flüssiger als auch in gasförmiger Form vorliegen kann, wobei ein Duftstoff von einem Geruchssensor erfasst und identifiziert werden kann.

Der Erfindung liegt die Erkenntnis zu Grunde, dass die Anwendung eines Teststreifens oder eines anders ausgestalteten Speichelsammlers öfters mühselig ist, weil zu wenig Speichel dem Mundraum des Probanden entnommen werden kann. Die Erfindung hat dieses Problem nicht nur erkannt, sondern im gleichen Schritt auch gelöst. Der Proband springt auf den Duftstoff an, und die abgesonderte Speichelmenge im Mund des Probanten erhöht sich nahezu sofort. Dies ermöglicht eine einfache und sichere Sammlung des Speichels. Dadurch wird die Gefahr der Beeinträchtigung der in vitro diagnostischen Tests durch eine falsche oder nicht ausreichende Sammlung der Speichelmenge vermieden.

Es ist zum einen denkbar, dass der Duftstoffträger ein separates Bauelement ist, welches mit dem Speichelsammler ein Kit bildet. Diese können beispielweise gemeinsam verpackt sein und auch ursprünglich aneinander befestigt sein. Das separate Bauelement des Duftstoffträgers lässt sich in diesem Fall jedoch vom Bauelement des übrigen Speichelsammlers trennen und separat handhaben. Auf diese Weise ist es möglich, den Duftstoffträger unmittelbar vor die Nase des Probanden zu halten, um die Speichelerzeugung anzuregen.

Alternativ lässt sich vorstellen, dass der Duftstoffträger am Speichelsammler fest angeordnet ist, auch einstückig mit dem Speichelsammler, wobei ein Duftbereich einen Abstand zu einem Speichelbereich aufweist. Dies macht die Handhabung des Speichelsammlers leichter, da nur ein Bauelement vorliegt.

Es wird vorgeschlagen, dass zwischen dem Duftbereich ein Abstand zum Speichelbereich von mehreren Zenitmetem vorliegt. Der Duftbereich ist derjenige Bereich, in welchem die Duftstoffe angeordnet sind, während der Speichelbereich derjenige Bereich ist, welcher dazu eingerichtet ist, den Speichel des Probanden aufzunehmen. Der Vorteil der getrennten Anordnung am Speichelsammler ist darin zu sehen, dass die Duftstoffe nicht in den Mundraum des Probanden gelangen, wo sie eventuell die späteren Testergebnisse verfälschen könnten.

Daher wird vorgeschlagen, dass der Duftbereich einen Abstand zu einem Mundbereich aufweist, insbesondere mit einer Verjüngung zwischen dem Mundbereich und dem Duftbereich. Eine Verjüngung wird den Probanden dazu veranlassen, die Lippen beim Schließen des Mundes in diesen Bereich zu legen, sodass der Duftbereich außerhalb der Lippen verbleibt.

Es ist auch denkbar, dass zwischen dem Duftbereich und dem Mundbereich eine Aufweitung des Speichelsammlers erfolgt. In diesem Fall wird der Proband meist seine Lippen maximal bis zur Aufweitung vorschieben und den Mund dort schließen. Eine Aufweitung macht zwar den erforderlichen Platz zum Aufbewahren der Speichelsammler größer. Andererseits wird es einer dritten Person, die dem Probanden den Speichelsammler in den Mund legt, angenehmer erscheinen, wenn die eigenen Finger vor dem Speichel des Probanden durch eine Aufweitung geschützt sind.

Hinsichtlich der konkreten Ausgestaltung des Duftstoffträgers sind fast keine Grenzen gesetzt. Es wird jedoch vorgeschlagen, dass der Duftstoffträger flächig ausgebildet ist. Dies ist zum einen relativ kompakt auf einen meist streifenförmigen Speichelsammler anzuordnen. Zum anderen hat eine Fläche eine relativ kontrollierbare Absonderungsrichtung für Duftstoffe, die etwa senkrecht zur Flächenerstreckung liegt.

Als ein flächiger Duftstoffträger kann beispielsweise eine Etikett verwendet werden, insbesondere ein Klebeetikett oder ein ansonsten haftendes, bevorzugt leicht ablösbares Etikett.

Für eine Ausbildung des Duftstoffträgers direkt am Speichelsammler wird insbesondere vorgeschlagen, dass der Duftstoff dort als eine Beschichtung aufgebracht ist.

In einer bevorzugten Ausführungsform weist der Duftstoffträger eine mechanische Freisetzeinrichtung für den Duftstoff auf. Hiermit kann gewährleistet werden, dass der Duftstoff gezielt zum Geruchssinn des Probanden gefördert wird. Insbesondere wird es ermöglicht, den Duftstoff nur bei Bedarf zu aktivieren. Auch dies führt zu einer Erhöhung der Ergebnisqualität.

Die mechanische Freisetzeinrichtung kann eine Sollbruchstelle aufweisen. Es sei insbesondere daran gedacht, den Duftstoff unter einer Hülle am Speichelsammler oder im separaten Duftstoffträger aufzubewahren, wobei diese Hülle durch eine mechanische Belastung zerstört werden kann oder zumindest eine Öffnung freigibt. Durch diese Öffnung kann der Duftstoff entweichen, sodass er vom Probanden wahrgenommen werden kann und die gewünschte Wirkung hinsichtlich des erhöhten Speichelflusses auslöst.

Bei einer Sollbruchstelle sei zum einen daran gedacht, dass die mechanische Widerstandsfähigkeit an einer Stelle, nämlich an der Sollbruchstelle, so weit herabgesetzt ist, dass bei einem Mindestmaß an Kraft, insbesondere Zugkraft, oder Biegemoment genau dort die Werkstofffestigkeit überschritten wird und ein Riss auftritt, der irreversibel ist. Es sei jedoch auch eine Ausbildung in Betracht gezogen, bei welcher sich eine Öffnung zeigt, welche sich bei Ablassen der Kraft wieder schließt. So ist ohne Weiteres vorstellbar, dass sich eine Öffnung in einer gewölbten Kunststoffhülle bei Aufbringen eines Biegemoments zeigt, während nach Entfernen des Biegemoments die Hülle die ursprüngliche, geschlossene Form wieder annimmt.

Eine Sollbruchstelle ist nicht nur ein sehr kostengünstiges Mittel, um den Duftstoff des Speichelsammlers bei Bedarf freigeben zu können, beispielsweise durch Ziehen am Speichelsammler, durch Tordieren des Speichelsammlers, durch Abziehen eines Abdeckstreifens auf dem Speichelsammler oder durch Aufschrauben einer Abdeckung am Speichelsammler. Vielmehr ist eine Sollbruchstelle auch sehr kompakt ausführbar.

Alternativ und kumulativ wird vorgeschlagen, dass die mechanische Freisetzeinrichtung einen Zerstäuber aufweist. Ein Zerstäuben des Duftstoffs kann zwar bereits dadurch geschehen, dass die Sollbruchstelle sprungartig versagt und dabei hohe Beschleunigungen und Geschwindigkeiten annimmt, die - wenn auch im mikroskopischen Maßstab - den Duftstoff vom Speichelsammler fortschleudern können. Ein Zerstäuber, insbesondere mit einer Düse, kann die Ergebnisse jedoch sehr stark verbessern.

Überraschend gute Probemengen an Speichel werden erreicht, wenn der Duftstoff einen Fruchtduft aufweist. Düfte von Früchten haben bei Untersuchungen des Erfinders hervorragende Ergebnisse geliefert, wenn diese oder eine Mischung von Düften mit Fruchtdüften zu den Geruchssensoren der Probanden geführt wurden. Insbesondere sei an saure, bittere oder süße Früchte gedacht, beispielsweise Zitrone, Pampelmuse oder Apfel.

Nach einem zweiten Aspekt der Erfindung löst die gestellte Aufgabe ein Verfahren zum Herstellen eines Speichelsammlers für eine Diagnose anhand des Speichels, wobei einem Kunststoff ein Duftstoff beigemengt wird.

Probensammler werden auch in der bisher bekannten Form oft aus Kunststoffen gefertigt. Dies erlaubt eine Beimengung eines Duftes während der Herstellung des Probensammlers, also insbesondere bei Kunststoff. Die Probensammler können hierdurch mit nur geringen Modifikationen in bereits bestehenden Herstellungsanlagen gefertigt werden.

Insbesondere ist sogar vorstellbar, dass einem Kunststoffgranulat Kapseln mit dem Duftstoff untergemengt werden. Dies ermöglicht bei geeigneter Gestaltung die Verwendung der bisherigen Produktionsanlagen ohne Änderung. Die Änderung spielt sich lediglich in den zugeführten Werkstoffen ab.

Nach einem dritten Aspekt der Erfindung löst die gestellte Aufgabe ein Verfahren zum Sammeln von Speichel an einem Probanden für eine Diagnose anhand des Speichels, bei welchem dem Probanden ein Mundbereich mit einem Speichelbereich in den Mund eingeführt wird, wobei ein Duftstoffträger in die Nähe der Nase des Probanden gebracht wird.

Es wurde bereits erläutert, dass der Duftstoff nicht nur die Menge des abgesonderten Speichels erhöht, sondern zusätzlich durch das Aussetzen der Testperson mit dem Duft außerhalb des Mundes die Speichelprobe frei von Kontaminierung bleibt.

Die Erfindung wird nachstehend anhand zweier Ausführungsbeispiele unter Bezugnahme auf die Zeichnung näher erläutert. Funktional gleiche Bauelemente können gleiche Bezugsziffern tragen.

Es zeigen
- Figur 1: schematisch eine Draufsicht auf einen Speichelsammler mit einer fest angebrachten Duftfläche,
- Figur 2: den Speichelsammler aus Figur 1 schematisch im Einsatz an einem Probanden und
- Figur 3: schematisch einen alternativen Speichelsammler mit einem separatem Duftetikett.

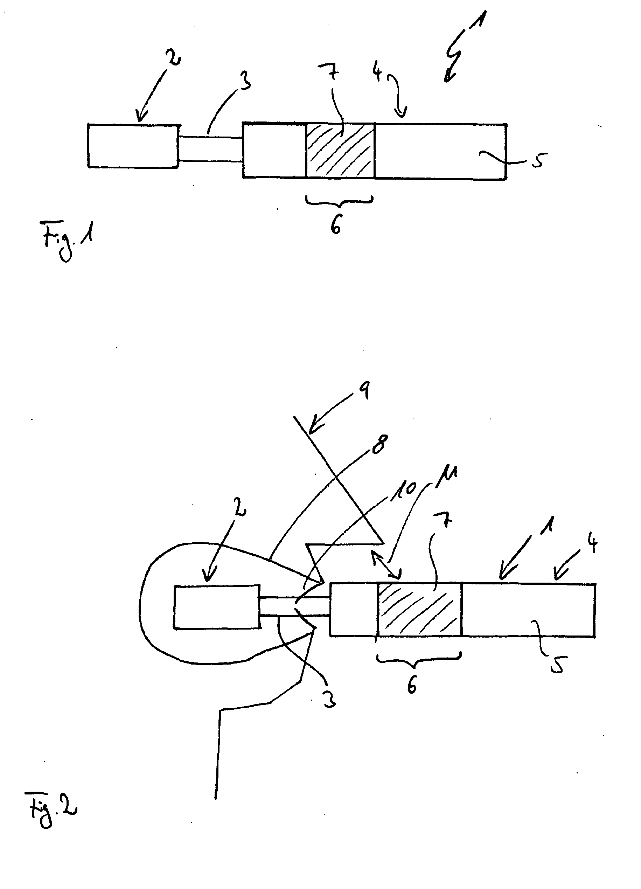

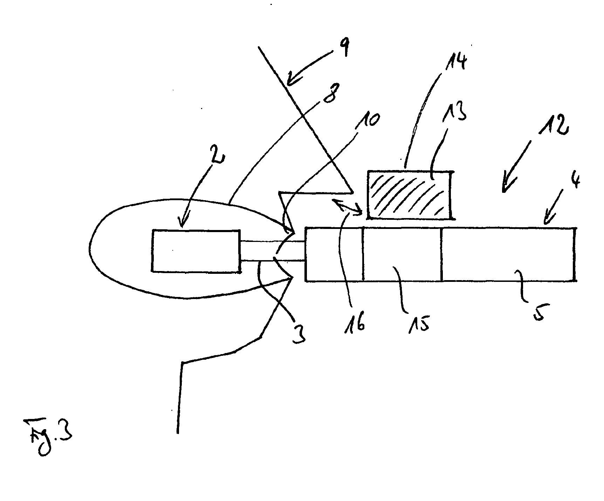

Der Speichelsammler 1 in den Figuren 1 und 2 hat im Wesentlichen die Form eines flachen Streifens mit einem Mundbereich 2, einer sich daran anschließenden Verjüngung 3 und einem wieder aufgeweiteten flächigen Streifen 4. Der flächige Streifen 4 weist zum einen einen freien Endbereich 5 auf, an welchem der Speichelsammler 1 gefahrlos gegriffen werden kann. Zum anderen ist - eher zur Verjüngung 3 hin orientiert - in einem Duftbereich 6 eine Beschichtung 7 mit einem Duftstoff (nicht dargestellt) vorgesehen.

Im Einsatz des Speichelsammlers 1 wird dieser mit dem Mundbereich 2 in eine Mundhöhle 8 eines Probanden 9 eingeführt, sodass sich Lippen 10 (exemplarisch dargestellt) an der Verjüngung 3 des Speichelsammlers 1 schließen. Auf dem Mundbereich 2 ist ein Speichelbereich (nicht dargestellt) angeordnet, der dazu geeignet ist, den Speichel des Probanden 9 aufzunehmen und für die Reaktion vor Ort oder später aufzubewahren.

Der Duftstoff hat vom Duftbereich 6 nur eine sehr kurze Distanz 11 bis zu einer Nase des Probanden 9 zurückzulegen, sodass der von dort entweichende Luftstoff den Probanden in einer hohen Konzentration erreicht, was den Speichelfluss in der Mundhöhle 8 anregt.

Der auf diese Weise ohne Kontaminierung mit Duftstoff gewonnene Speichel des Probanden 9 kann dazu verwendet werden, diagnostische Krankheiten, diagnostische Stoffe, pharmakologische Stoffe, diagnostische Kontaminierungen, pharmakologischen Missbrauch von Stoffen und Drogenkonsum nachzuweisen.

Der alternativ ausgeführte Speichelsammler 12 in Figur 3 ist weitgehend gleich aufgebaut.

Ein Duftbereich 13 ist jedoch auf einem vom flächigen Streifen 4 des Speichelsammlers 12 lösbaren Etikett 14 angeordnet. Das Etikett 14 dient als Duftstoffträger und ist somit ein separates Bauelement, welches gemeinsam mit dem übrigen Speichelsammler 12 ein Kit zum Sammeln von Speichel eines Probanden 9 bildet.

Am flächigen Streifen 4 des Speichelsammlers 12 ist ein Anbringbereich 15 für das Etikett 14 vorgesehen. Das Etikett 14 kann somit vor dem Einsatz am flächigen Streifen 4 transportiert werden.

Zum Einsatz des Speichelsammlers 12 wird wiederum der Mundbereich 2 in die Mundhöhle 8 des Probanden 9 eingeführt. Davor oder danach wird das Etikett 14 jedoch vom Speichelsammler abgezogen. Das Etikett 14 ist hierzu auf einer Unterseite mit einem leicht lösbaren Klebstoff ausgestattet. Es besteht selbst aus einer relativ dicken Folie mit einer spröden Oberflächenkonsistenz. Beim Abziehen des Etiketts 14 vom Anbringbereich 15 wird das Etikett 14 gebogen. Hierdurch treten zahlreiche Mikrorisse in der Oberfläche und somit im Duftbereich 13 auf, woraufhin ein unterhalb der Oberfläche vormals geschützt liegender Duftstoff freigesetzt wird. Das Etikett 14 verströmt nun einen starken Duft.

In diesem Zustand wird es möglichst zügig auf eine kurze Distanz 16 zur Nase des Probanden 9 geführt.

## Patentansprüche

1. Speichelsammler (1, 12) für eine Diagnose anhand des Speichels, ***dadurch gekennzeichnet, dass*** ein Duftstoffträger (7, 14) für einen Duftstoff vorgesehen ist, der einen Probanden (9) zu erhöhtem Speichelfluss veranlasst.

2. Speichelsammler nach Anspruch 1, ***dadurch gekennzeichnet, dass*** der Duftstoffträger (14) ein separates Bauelement ist, welches mit dem Speichelsammler (1, 12) ein Kit bildet.

3. Speichelsammler nach Anspruch 1, ***dadurch gekennzeichnet, dass*** der Duftstoffträger (7) am Speichelsammler (1, 12) fest angeordnet ist, wobei ein Duftbereich (6) einen Abstand zu einem Speichelbereich aufweist.

4. Speichelsammler nach einem der vorhergehenden Ansprüche, ***dadurch gekennzeichnet, dass*** der Duftbereich (6, 13) einen Abstand zu einem Mundbereich (2) aufweist, insbesondere mit einer Verjüngung (3) zwischen dem Mundbereich (2) und dem Duftbereich (6).

5. Speichelsammler nach einem der vorhergehenden Ansprüche, ***dadurch gekennzeichnet, dass*** der Duftstoffträger (7, 14) eine mechanische Freisetzeinrichtung für den Duftstoff aufweist.

6. Speichelsammler nach Anspruch 5, ***dadurch gekennzeichnet, dass*** die mechanische Freisetzeinrichtung eine Sollbruchstelle aufweist.

7. Speichelsammler nach Anspruch 5 oder 6, ***dadurch gekennzeichnet, dass*** die mechanische Freisetzeinrichtung einen Zerstäuber aufweist.

8. Speichelsammler nach einem der vorhergehenden Ansprüche, ***dadurch gekennzeichnet, dass*** der Duftstoff einen Fruchtduft aufweist.

9. Verfahren zum Herstellen eines Speichelsammlers (1, 12) für eine Diagnose anhand des Speichels, insbesondere zum Herstellen eines Speichelsammlers (1, 12) nach einem der Ansprüche 1 bis 8, ***dadurch gekennzeichnet, dass*** einem Kunststoff ein Duftstoff beigemengt wird.

10. Verfahren zum Sammeln von Speichel an einem Probanden für eine Diagnose anhand des Speichels, bei welchem dem Probanden (9) ein Mundbereich (2) mit einem Speichelbereich in den Mund eingeführt wird, ***dadurch gekennzeichnet, dass*** ein Duftstoffträger (7, 14) in die Nähe der Nase des Probanden (9) gebracht wird.
